(19) Europäisches Patentamt

European Patent Office

Office européen des brevets

(11) **EP 0 781 527 B1**

(12) **EUROPEAN PATENT SPECIFICATION**

(45) Date of publication and mention
of the grant of the patent:
**08.10.2003 Bulletin 2003/41**

(51) Int Cl.$^7$: **A61B 5/00**, G02B 6/36

(21) Application number: **96660092.6**

(22) Date of filing: **27.11.1996**

(54) **Non-invasive optical sensor**

Nichtinvasiver optischer Sensor

Capteur optique non-invasif

(84) Designated Contracting States:
**DE FR GB IT SE**

(30) Priority: **29.11.1995 FI 955758**

(43) Date of publication of application:
**02.07.1997 Bulletin 1997/27**

(73) Proprietor: **Instrumentarium Corporation**
**00510 Helsinki (FI)**

(72) Inventors:
• **Weckström, Kurt Peter**
**02170 Espoo (FI)**

• **Ristolainen, Kimmo Juhani**
**00970 Helsinki (FI)**

(74) Representative: **Laako, Tero Jussi**
**Berggren Oy Ab**
**P.O. Box 16**
**00101 Helsinki (FI)**

(56) References cited:
**DE-B- 2 517 129**     **US-A- 3 123 066**

• **YOSHIYA I. ET AL: 'Spectrophotometric
monitoring of arterial oxygen saturation in the
fingertip' MED. & BIOL. ENG. & COMPUT. vol. 18,
1980, pages 27 - 32**

**Description**

The present invention relates to an optical sensor according to independent claims 1 and 6. The invention relates also to the use of such a sensor to measure oxygen saturation in a patient's blood.

Pulseoximeters are capable of measuring the degree of oxygen saturation in a patient's blood non-invasively and continuously. The term non-invasive indicates that the patient is not subjected to subcutaneous penetration by any physical means but the measuring is effected by means of radiation and other than that the procedure occurs externally of the body. This type of monitoring of blood oxygen saturation is quite common today and is one of the monitoring parameters required in many applications. The measurement is optical and based on various absorption characteristics of red and infrared light in blood hemoglobin. The principles of pulseoximetry are disclosed e.g. in Patent publication JP-53-26437. This reference describes a pulsoximeter sensor which comprises a wide-band thermic radiation source and two optical detectors fitted with a bandpass filter for expressing various wavelengths. At present, the pulseoximeter sensors include two light sources, which are typically light emitting diodes (LED), and a single detector, and the radiation sources are operated alternately in synchronism. In each case, the radiation sources and detectors are included in the sensor itself near a target to be measured and from the sensor extend electrical cables to a control monitor. Usually, the patient experiences no problems when using high-efficiency light sources with a low power consumption. However, the signal-to-noise ratio may sometimes suffer, particularly if for some reason it is desirable to use wavelengths other than those provided by said light sources. In order to achieve as powerful a signal as possible, the light sources can be supplied with a lot of electric power. This may sometimes lead to the situation that, as a result of a higher input of power, the heat generated by light sources affects a target to be measured and causes even burns. Thus, in practice, there is sometimes no choice but to settle for quite a poor signal when sufficient light cannot be produced without coincident heat generation. Neither can the above-described conventional solution be used for example during the course of currently more and more popular magnetic imaging of a patient or while operating some other powerful electromagnetic source. In these conditions, the electrical cable of a sensor would act as an antenna for interferences originating from a control monitor and these interferences would upset the magnetic images. Also other metallic parts at the sensor end disturb a magnetic imager and distort the imaging result. Also in the imaging field, the electric conductor of a sensor may be induced with currents sufficiently strong for a patient to sustain burns or to break down the pulseoximeter equipment. In order to secure its immaculate operation in magnetic imaging environment, the oximeter sensor must not include any metallic components at all and, thus, all normal electronics must also be excluded.

The pulseoximeter sensors compatible with magnetic imaging environment are typically designed with fiberoptics. Hence, the light sources and detector are spaced away from an actual site to be imaged. The light supply is led to and from a site to be measured along optical fibers. In practice, the fiberoptic cable is a bundle of fibers, comprising a plurality of thin optical fibers and having typically a diameter of 1-3 mm. In order to develop a clinically useful sensor, it is necessary in many cases to bring the bundles of optical fibers to the sensor end in the direction at least roughly parallel to sensor housings. This requires that the light supply be deflected by about 90° inside the sensor housing. The light supply can be deflected by bending the optical fibers to a 90° angle, as disclosed in the publication MEDICAL & BIOLOGICAL ENGINEERING & COMPUTING, Vol. 18 /1980 pp. 27 - 32: Yoshiya, Shimada, Tanaka - "Spectrophotometric monitoring of arterial oxygen saturation in the fingertip". In terms of production, however, this solution is difficult to carry out with sufficiently large bundles of fibers and with a sufficiently small bending radius. The cited glass fibers are easily broken upon bending. Likewise, some of the light supply manages to escape out of the fibers at a sharp curve formed in the fibers.

Other representative fiberoptic designs for a pulseoximeter sensor are described in publications US-5,279,295 and WO-92/21281. In both solutions, the end of a fiber bundle is more or less bent for guiding a light signal to and from a target. In each case, the object of measuring is a patient's finger. In publication US-5,279,295, the monitoring is based on the reflection of light from the finger while publication WO-92/21281 discloses a more conventional solution, wherein the finger is transilluminated. In publication WO-92/21281, the material of a fiberoptic radiation guide is determined to be plastics, i.e. the question is about a plastic fiber, which is probably a little more resistant to bending than glass fiber. However, a drawback in the plastic fiber is that, as pointed out in the publication, its transmittance does not extend very deep into the infrared range, which prevents the use of wavelengths that would be optimal in terms of measuring and also the use of best possible radiation emitting diodes (LED). In these solutions, as well, some radiation may escape out of the sharp fiber curve. The described solutions provide a signal which is poorer than what is achieved if a straight bundle of fibers were orthogonal to the target. Furthermore, the bending of a bundle of fibers is an expensive approach in terms of productivity. A bundle of fibers in the proximity of a target represents a relatively small surface area. A result of this is that the sensor is sensitive to a so-called motion-related artefact. These result either directly from the movements of a target relative to the sensor or from volumetric changes, i.e. absorbency changes, introduced in venous blood by external causes. The harmful effect is further enhanced by the fact that the numerical fiber aperture is relatively small. Normally, a bundle of fibers accepts light rays within the range of +/- 40° or at best +/-60° with respect to the fiber axis. This is modest with respect to a conventional sensor, wherein the source radiates in principle +/-90° and also the detector accepts the same amount.

In publication DE-25 17 129 a photoelectric pulse receiver with fiber optics is disclosed. The receiver comprises a radiation source and a detector remote from the body of a patient, and a fiber optical sensor having an application surface at the proximal end thereof, which application surface is positioned on the body of the patient and the radiation is guided via a fiber optical sensor. According to this publication the application surface of this sensor is larger than the cross-section of the sensor, evidently the cross-section of the bundle of the optical fibers, is positioned essentially parallel with the longitudinal axis of the sensor and is provided with sections for said source and said detector. In all embodiments shown in this publication the optical fibers are bent and in most cases at angles of 90° in order to get the application surface to be positioned parallel with the sensor length. This bending of the fibers very often causes breaking thereof or at least surface cracks. The larger application surface is obtained by separating the end portions of the individual fibers from each other and positioning these over a larger area. In this case the aperture of the application surface is exactly the same as the aperture the bundle of the optical fibers in the sensor, because the radiation transmitting cross-sectional area is not altered in any way. The large application surface is mainly composed of the dark spacings between the individual fibers.

The alternative constructions to form said larger application surface utilize a protective layer with scattering and reflective properties at the ends of the fibers.

The direction of light supply can also be deflected by means of some external structure, for example a reflective mirror surface. The reflective mirror surface is created e.g. by metallizing a shiny plastic surface. However, a metallic mirror is not capable of deflecting all radiation supply coming from the fibers. Similarly, a metallic mirror is invonvenient in coupling a sufficient amount of tissue-penetrated radiation with a second bundle of fibers and further with the detector. Another downside in a metallic mirror is a metallic layer included therein, which, as a result of evolving eddy currents, may warm up during magnetic imaging and expose the patient to burn hazards. Furthermore, as described above, a metallic layer may disturb magnetic imaging.

A first object of this invention is to provide a sensor, wherein at least one radiation source or at least one bundle of fibers can be used for supplying a target with radiation and/or radiation can be collected from a target to at least one detector or at least one bundle of fibers effectively, advantageously and compactly, which particularly means a high efficiency in the transmission of radiation. A second object of the invention is to provide a sensor which is not highly sensitive to a motion-related artefact. A third object of the invention is to provide a sensor, wherein the heat generated by light sources does not reach a target to be measured but, as a result of the configuration, can be led away therefrom which, if necessary, enables the use of high-power radiation sources. A fourth object of the invention is this type of sensor which does not restrict too severely the use of an otherwise desired infrared wavelength range.

The above-described drawbacks can be eliminated and the above-defined objects can be achieved by means of a sensor of the invention, which is characterized by what is set forth in the characterizing clauses of claims 1 and 6 and by the use of such a sensor, said use being characterized by what is set forth in the characterizing clause of claim 14.

In an apparatus of the invention, the light supply can be effectively collected and, if necessary, deflected inside a sensor housing by means of a diffusively reflecting surface. Measuring tests have indicated that, when using this diffusively reflecting surface of the invention in association with optical fibers, the resulting signal is even substantially stronger than that produced by means of target-attached straight or bent fibers. This apparent paradox is explainable by the fact that it is possible to employ larger target areas, as will be manifested hereinbelow. The solution is beneficial since the required materials are inexpensive and it occupies little space since the bundle of fibers need not be bent. In addition, it is possible to employ reasonably priced standard straight fiber bundles instead of expensive and damage-prone special designs. In the proximity of a target to be measured, the light apertures are sufficiently large, such that light can be delivered to the target consistently and also collected therefrom consistently over an area larger than the fiber diameter. By virtue of this, the sensitivity to a motion-related artefact will be insignificant. Conditions permitting, a diffusively reflecting surface of the invention can be used without optical fibers in a sensor equipped with light sources and a detector. Thus, it is preferred that the side facing light sources be provided with a diffusively reflecting surface. This way the light sources are brought further away from a target to be measured and the heat generated thereby is easily carried away from the target.

The invention will now be described in detail with reference made to the accompanying drawing figures.

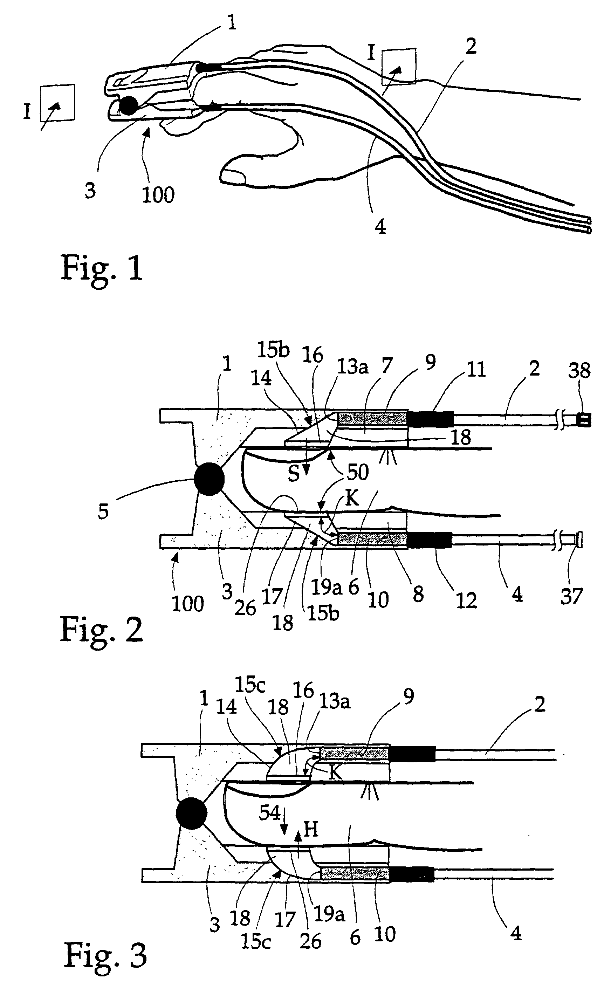

Fig. 1 is a plan view of a typical embodiment for a sensor of the invention, which is fitted with optical fibers for the transfer of radiation and a measuring signal and, thus, for spacing a radiation source and detectors from a sensor itself and which is designed to be fitted on a finger and to operate on a finger-penetrating measuring technique.

Fig. 2 is a lengthwise cross-section along a plane I-I in fig. 1, showing a first embodiment for a sensor of the invention, employing fiberoptics for spacing radiation sources and a detector from the actual sensor.

Fig. 3 is a lengthwise cross-section along a plane I-I in fig. 1, showing a second embodiment for a sensor of the invention, employing fiberoptics for spacing radiation sources and a detector from the actual sensor.

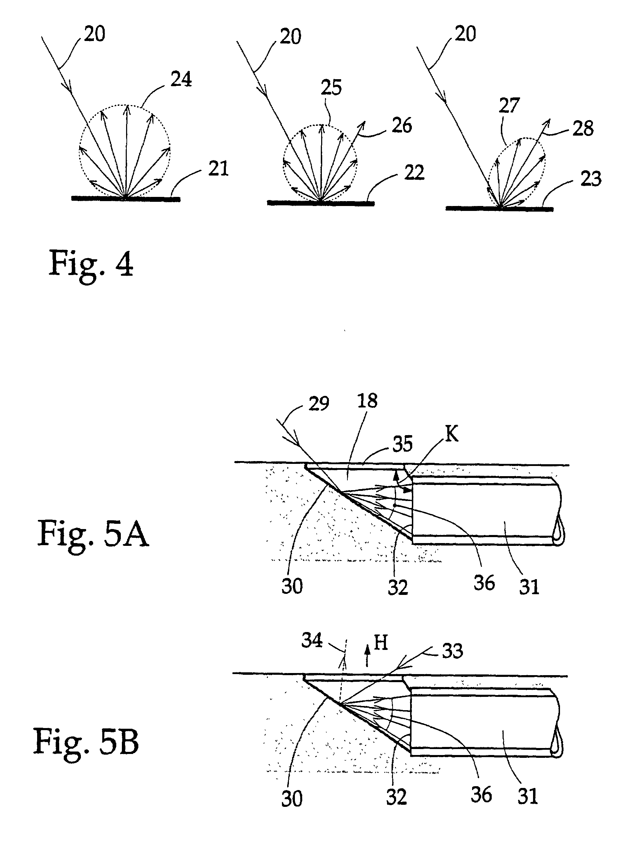

Fig. 4 shows graphically the reflection characteristics of diffusively reflective surfaces of the invention.

Fig, 5A and 5B depict the behaviour of a diffusively reflective surface of the invention with radiation arriving thereon from various angles of incidence.

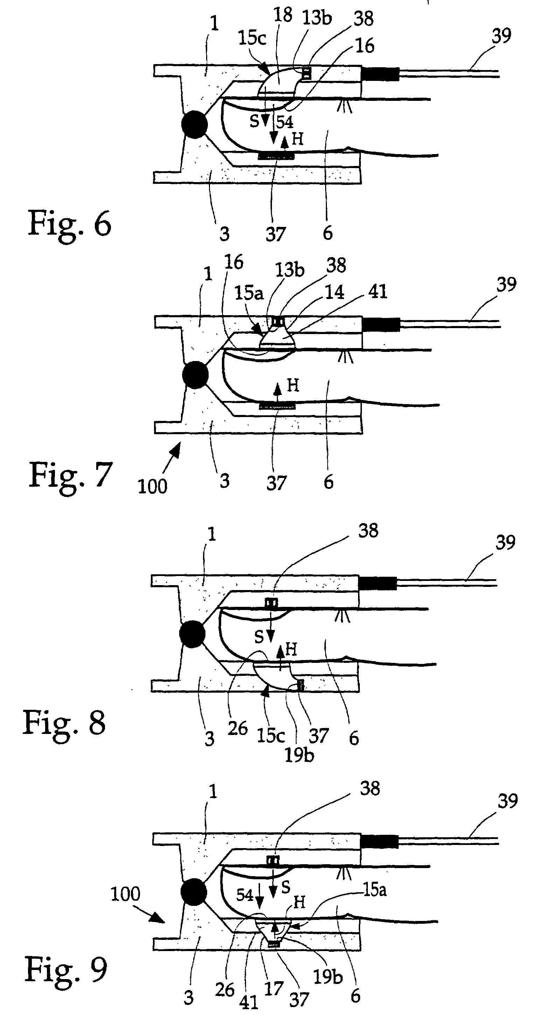

Fig. 6 is a lengthwise cross-section, in a view similar to figs. 2 and 3, showing a third embodiment for a sensor of the invention, wherein light sources are located remotely from a target to be measured and electric current is brought to and a signal delivered from the sensor along electric conductors.

Fig. 7 is a lengthwise cross-section, in a view similar to figs. 2 arid 3, showing a fourth embodiment for a sensor of the invention, wherein light sources are located remotely from a target to be measured and electric current is brought to and a signal delivered from the sensor along electric conductors.

Fig. 8 is a lengthwise cross-section, in a view similar to figs. 2 and 3, showing a fifth embodiment for a sensor of the invention, wherein a detector is located remotely from a target to be measured and electric current is brought to and a signal delivered from the sensor along electric conductors.

Fig. 9 is a lengthwise cross-section, in a view similar to figs. 2 and 3, showing a sixth embodiment for a sensor of the invention, wherein a detector is located remotely from a target to be measured and electric current is brought to and a signal delivered from the sensor along electric conductors.

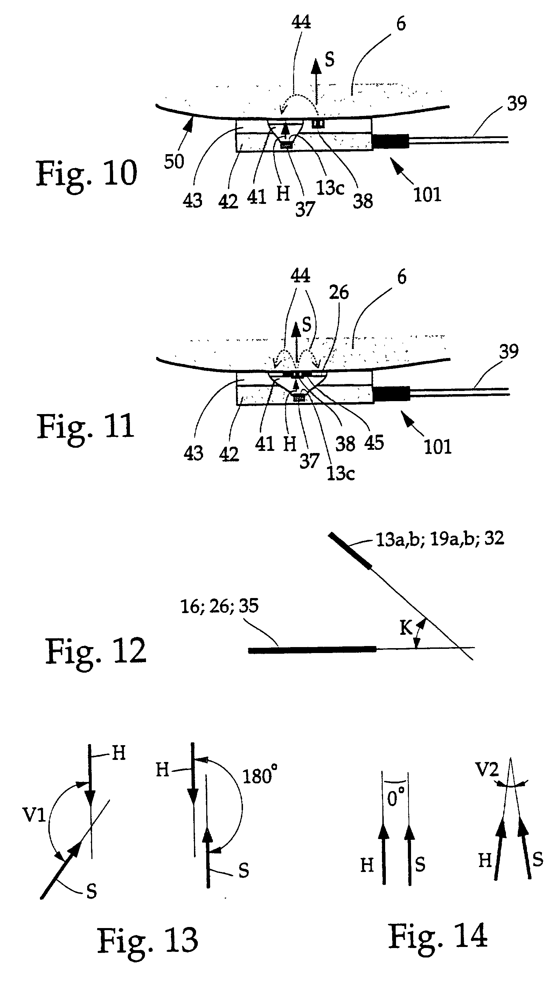

Fig. 10 is a lengthwise cross-section, corresponding to the view of figs. 2-9, showing a seventh embodiment for a sensor of the invention, wherein a detector is located remotely from a target to be measured and electric current is brought to and a signal delivered from the sensor along electric conductors and which is designed to be placed on the surface of a patient's body for a measuring technique effected by reflecting from a patient's tissue.

Fig. 11 is a lengthwise cross-section, in a view similar to figs. 2 and 3, showing an eighth embodiment for a sensor of the invention, wherein a detector is located remotely from a target to be measured and electric current is brought to and a signal delivered from the sensor along electric conductors and which is designed to be placed on the surface of a patient's body for a measuring technique effected by reflecting from a patient's tissue.

Fig. 12 shows in principle the relative position of those radiation-transmissive end walls whereby the measuring radiation arrives on a diffusively reflective surface of the invention.

Fig. 13 shows in principle the first relative directions of the principal radiating direction for a radiation source assembly of the invention and the principal sensitivity direction for a detector assembly of the invention, corresponding to the embodiments of figs. 2-9.

Fig. 14 shows in principle the second relative directions of the principal radiating direction for a radiation source assembly of the invention and the principal sensitivity direction for a detector assembly of the invention, corresponding to the embodiments of figs. 10 and 11.

A typical pulseoximeter sensor is attached to a fingertip as shown in fig. 1. In this application, the expressions radiation and light are used for representing the same thing, i.e. an electromagnetic radiation regardless of a wavelength. In this embodiment, the radiation or light arrives in a top sensor portion 1 along a fiberoptic cable 2 and a light 54 having passed through the finger is collected in a bottom sensor portion 3 for delivering it therefrom along a fiberoptic cable 4 to a detector. In commercially available sensors, the light source generally comprises two diodes (LED) emitting at different wavelengths and the detection of light is generally effected e.g. by means of a single silicon detector, since the applied wavelengths remain within its sensitivity range. Thus, the two radiating diodes (LED) are operated alternately in synchronized manner at an appropriate frequency, whereby signals produced at various wavelengths are distinguishable from each other. There is nothing to exclude the use of even several wavelengths as long as, for example, the present optical fiber is capable of transmitting the same and also other optical components operate in a manner compatible therewith. It is also possible to employ other types of radiation sources, such as thermic emitters or lasers. It is also possible to employ two wide-band radiation sources and two detectors having sensitivies which are adapted, e. g. by means of filters transmissive to narrow wavelength bands, to comply with desired wavelengths. In terms of this invention, neither the wavelength of applied radiation nor the width of a wavelength band nor the number of radiation sources and detectors nor the type of radiation sources and detectors are essential features. In addition to a fingertip, the pulseoximeter sensor can be attached for example to the ear, the palm of a hand, or a toe. In that case, the sensor has a different appearance but the diffusively reflective surfaces of this invention function exactly the same way regardless of a measured target. Therefore, it is primarily a finger sensor that will be studied hereinbelow.

A finger sensor 100 as shown in fig. 1 is depicted e.g. in fig. 2 in a lengthwise section. The sensor 100 has its top section 1 and bottom section 3 hinged at 5 in such a manner that both sections of the sensor are capable of opening and closing around a finger 6. Generally, the sensor remains stationary by means of a spring force and a friction developed by pads 7 and 8.

Fiberoptic cables 2 and 4, having optical diameters typically in the order of 1-3 mm, are connected to the top and bottom sensor sections 1 and 3 by means of a conventional ferrule 9 and 10 for holding individual fibers in a single bundle and by means of a dewaterer 11 and 12 for providing a durable assembly. The ferrules 9 and 10, like generally all other components in the sensor, are made of materials other than metal if the intension is to employ the sensor in an electromagnetic field. In order to provide a clinically useful sensor, the fiberoptic radiation conductors 2 and 4 arrive in the sensor 100 more or less in the direction of a finger and in general terms the fiberoptic cables arrive in the sensor principally in the direction parallel to the external surface of a target to be measured, in this case an external finger surface 50. Thus, for example in a finger sensor, the optical fibers may arrive in the sensor from the direction of the palm or from the direction of the fingertip or in a direction crosswise to the length of the finger. In a sensor connectable to the earlobe, the optical fibers arrive typically in the direction of the earlobe, and in a sensor to be fitted inside the ear, in the direction of the external auditory canal. The use of fiberoptic radiation conductors enables all metallic and electric components to be placed remotely from the actual sensor 100, such as in connection with the actual measuring monitor or in some converter between sensor and monitor. The distance is selected to be sufficient for any given application, such that the pulseoximeter sensor does not cause trouble or be troubled itself. When using a diffusively reflective surface of the invention, the optical radiation conducting fiber may comprise a glass fiber which is well transmissive even to quite long-wave infrared radiation since, in a solution of the invention, the fiber need not be bent for bringing the radiation to a desired angle, i.e. generally to an orthogonal direction, relative to the external surface 50 of the target 6.

The light, arriving from a radiation source 38 along a fiber cable 2, exits from the fiber end at a point provided with an inner end wall 13a included in a radiation transfer section of the invention, as a beam of scattering light having an opening angle around the centre axis typically of +/-40° and with wide-angle fibers +/-60°. A small portion of this light may fall on the external surface 50 of the finger 6 but most of it falls on a diffusively reflective surface 14 of the invention, which is included in a cavity 18 and has a funnel-like shape as shown in the figure and opens towards the target 6. Preferably, the inner end wall 13a of the cavity 18 has a surface area which is in the same order as the cross-sectional area of the bundle of fibers and the surface area of an outer end wall 16 in the direction of at least roughly parallel to the finger surface 50 is larger than that. Thus, the cavity 18 diverges towards a target. The radiation arrives at the transfer section by way of the inner end wall, progresses by way of the diffusively reflective surface 14 and penetrates into a target to be measured through the outer end wall. Thus, depending on the fiber 2, the inner end wall 13a may have a diameter of e.g. 1-3 mm and a preferred diameter for the outer end wall 16 is for example about 7 mm, which enables the scattering of light over a larger surface area and the sensitivity to a motion-related artefact remains insignificant with no light escaping from source to detector without passing through a target. Also, a large surface area complies better with the pulseoximeter's calibrating curve used for determining the relationship with the oxygen content of blood as the local inhomogeneities of a target, such as bone-induced shadings or individual venous blood vessels, are not able to have as strong an effect on the measuring result as would be the case with a smaller outer-end-wall surface area. In terms of operation, the surface areas of the apertures may have any given ratio but, in practice, it is thus preferred that the aperture which opens towards a target to be measured be larger. In a typical case, the outer end wall has a surface area which is at least double with respect to that of the inner end wall but it can be even considerably larger, e.g. tenfold.

The exact configuration of the diffusively reflective surface 14 is not critical, either. It can be a slanting cone 15b, as in fig. 2, but some other configuration, such as an arched-surface funnel 15c shown in fig. 3, functions equally well. In the embodiment of figs. 5A-5B, the funnel can be an oblique wedge, whereby a diffusively reflective surface 30 is flat or a slanting cone or cylinder, said diffusively reflective surface 30 being concave towards the inside of a cavity 18. In the former case, the image-plane facing cross-section of the cavity is a rectangle and in the latter case it is a circle or an ellipse. If it is flat, the diffusively reflective surface should be preferably arranged at least in a roughly orthogonal attitude towards the bisector of an angle formed by the inner-end-wall normal and the outer-end-wall normal together, as shown e.g. in figs. 5A-5B. As for the arched funnel 15c, on the other hand, it is appropriate to arrange the inner end wall and the outer end wall in such a manner that the normals thereof intersect the diffusively reflective surface or a surface section or an extension thereof, in other words the normals of the end walls must be directed towards the diffusively reflective surface. The aperture of the outer end wall 16 of a funnel is preferably covered e.g. with a window for preventing dirt from finding its way into the cavity 18 and further onto the end surface of a bundle of fibers or onto the diffusively reflective surface. The window may be constructed e.g. of silicone or some other appropriate material. Another option is to fill the entire cavity 18 with a light transmitting material, for example with similar transparent silicone. This is not harmful for the proper function of a diffusively reflective surface. It is obvious that the inner and outer end wall can be sealed with windows made of an appropriate material and the cavity 18, 41 filled with an appropriate gas or liquid.

Having passed through the finger, the light is completely scattered and at the point of measuring each spot of the finger operates as a Lambertian source. Light is arriving from quite a wide range and the effective collection thereof on the endwall surface of the fiber or bundle of fibers 4 is not easy no matter which of the actually occurring diameter and opening angle is used for the fiber or bundle of fibers. Nevertheless, the fiber 4 should be capable of carrying to a detector 37 as much as possible of the radiation coming from a target to be measured. A diffusively reflective surface 17 similar to the surface 14 contributes even on the radiation collection side to an increased collecting efficiency, as pointed out hereinafter. In terms of its dimensions, it can be similar to the funnel surface 14 but it may also have some other configuration, particularly if the fiber bundles 2 and 4 are different from each other. Thus, the collection of radiation from a target 6 is effected by means of a similar type of cavity 18 as the supply of radiation to the target 6, said cavity 18 thus including an inner end wall 19a, by way of which the collected radiation transfers to the fiber 4, as well as an outer end wall 26, by way of which the radiation enters inside the cavity to be subsequently reflected from the diffusively reflective surface 17. The design can be totally analogous to the above-described radiation supplying cavity. Thus, the outer end wall 26 has a surface area which preferably exceeds that of the inner end wall 19a, typically the outer end wall has surface area which is at least double with respect to that of the inner end wall, but it may be even considerably larger, e.g. tenfold. In view of the actual operation, the apertures may have surface areas whose ratio to each other can be anything, as already described above. Also in this case, the end walls must have the normals thereof directed towards the diffusively reflective surface or an extension thereof, as explained above. Even in this case, the aperture in the outer end wall 26 of the funnel can be covered with transparent silicone or some other compatible material or the entire cavity 18 filled with this material without disturbing operation of the diffusively reflective surface.

In the above-described embodiments of figs. 2 and 3, in the embodiment of figs. 5A and 5B, as well as partially in the embodiments of figs. 6 and 8, the cavity 18 is provided with inner end walls 13a, 19a, 13b, 19b and 32 which form an angle K relative to the outer end wall 16, 26 and 35 of the cavity, said angle being within the range of 30°-100°, generally at least 45°, preferably within the range of 60°-95° and typically about 90°. This angle is explained further in fig. 12. In figs. 2-3, 5A-5B, 6 and 8, the angle K is essentially a right angle, but if so required by a particular application, it can be somewhat more than 90°, as point out above. This described angle configuration enables the passage of the fiberoptic radiation conductor 2, 4 to a sensor e.g. in the direction of the external surface 50 of a target or in some other appropriate direction without having to bend the optical fiber. Naturally, the inner end walls 13b and 19b can be perfectly or approximately parallel to the given respective outer end wall 16 and 26, as depicted in figs. 7 and 9 and to be subsequently described in more detail. In any event, between the inner end wall and the outer end wall is fitted a diffusively reflective surface of the invention. The outer end wall is set against the external surface of a target to be measured, whereby a slight gap is often left therebetween no matter if the question is about the side supplying radiation to a target or the side collecting radiation from a target. The inner end wall refers to a point of contact with an optical fiber or some other radiation conducting element or with radiation sources or a detector or a like. In theory, it is not absolutely necessary that a jacket of the cavity 18 be provided between the end walls to surround the end walls as well as the diffusively reflective surface although, in practive, the cavity and its jacket are normally necessary for avoiding external mechanical and physical and chemical drawbacks.

The qualities of a diffusively reflective surface are depicted in fig. 4. A ray of light 20 falls on various types of diffusively reflective surfaces 21, 22 and 23. The roughness of a surface and the optical characteristics of a material determine how the ray is reflected. It is of course desirable that the surface does not absorb the applied radiation and that the material is not so thin that the light is able to escape therethrough. The light may reflect directly from the surface or it may travel deeper in a diffusively reflective material prior to reflecting back. The surface 21 is a so-called Lambertian surface which completely scatters the light 20 fallen thereon. In that case, the roughness of a surface material or the size of the structural elements of a material is lesser than or in the same order as the light's wavelength. The light scatters in every direction such that the reflected radiation has an intensity distribution which follows a dotted line 24 (the distribution is represented by a sphere which is tangential with the reflective surface, the intensity vectors starting from tangential points and terminating at the ball surface). Such a surface appears equally bright from every direction and is theoretically the best possible scattering surface, i.e. a diffusively reflective surface. Such a surface is difficult to manufacture, but for example Labsphere, Inc. is marketing a material called Spectralon, having characteristics which are very close to those of the surface 21. The same type of material is also available as a coating. The materials and coatings have a reflectance of about 99 %, hence the absorption and transmission are insignificant. The surface 22 depicts a case in which a scattering section 25 is significant but in which also a mirror reflection 26 of the surface is visible. It is useful also in a sensor of the invention although the efficiency is slightly poorer than what is achieved by the surface 21. Several scattering materials belong in this category. For example, the commercially available silicone polymers, which are readily mouldable to a proper shape, may remain slightly shining over the surface thereof and, thus, some mirror reflection is inevitable. However, the mirror reflection of a surface has an intensity whose proportion is generally modest with respect to the diffusively reflected intensity, since the mirror reflection depends on the index of refraction of a material, which on silicone polymers is relatively low, and since the material is non-metallic. The surface 23 is rougher than the surfaces 21 and 22, as evidenced by the fact that an intensity distribution 27 is an ellipse or ellipsoid parallel to a mirror reflection 28. However, the scattering or ray-diffusing effect continues to be dominant. The ray-diffusing results from the fact that light reflects from various roughness patterns of a surface in directions other than the one in which the radiation would reflect from a common mirror surface and, hence, the intensity patterns resembles a scattering pattern. Generally, the practical diffusively reflective surface is a combination of all surfaces shown in fig. 4. Although non-metallic materials are in most cases used in diffusively reflective surfaces, it is not inconceivable, in principle, to employ a suitably surface-treated metal as well, if the particular application allows the use of metal in the sensor. A metal treated to be diffusively reflective may be the only solution when it is necessary to use extremely long wavelengths. However, this is not the case in a normal pulseoximeter sensor and, thus, the use of non-metallic materials is plausible.

Figs. 5A and 5B illustrate how the diffusively reflective surface operates as a light collecting system and why its use is preferred. In fig. 5A, a ray 29 arrives from a radial direction preferable in terms of mirror reflection from a diffusively reflective surface 30, which can be any of the surfaces 21, 22 or 23 depicted in fig. 4. Even after the mirror reflection, the ray would be likely to fall on the endwall surface of a bundle of fibers 31, which is in alignment with an inner end wall 32, since the bundle of optical fibers 31 accepts all angles 36 smaller than the opening angle of the fiber. In fig. 5B, on the other hand, a ray 33 arrives from such a V direction that the mirror reflection would take it completely out of the system as a ray 34. This is not preferred, although it is possible to presume that a portion of the ray, having reflected again from the surface of a target, arrives in the funnel, be it in a decisively weakened condition. Presuming subsequently that the surface 30 is totally diffusively reflective, i.e. a so-called Lambertian surface mentioned above, and performing a comparison between such a diffusively reflective surface of the invention and a bundle of fibers in direct contact with a target. Supposing further that the bundle of fibers 31 has a diameter of 2 mm in keeping with the diameter of the inner end wall 32 and that an outer end wall 35, i.e. an inlet aperture, included in the cavity 18 and opening towards the target, has a diameter of 7 mm. The operating principle of a diffusively reflective cavity is the same as that of an integrating sphere and, thus, it is possible at a sufficient accuracy to apply computation formulae relating to an integrating sphere. The cavity includes two apertures 32 and 35, as generally found in an integrating sphere. As an exception from the normal integrating sphere it is possible to accept the fact that some radiation advances directly from aperture 35 to aperture 32 without hitting the cavity wall, in other words, the aperture 35 is visible from the aperture 32. This is beneficial in terms of the invention as there will be no reflection losses. It is contemplated, however, that the entire radiation falls at least once on the diffusively reflective surface 30. A portion T2 of the intensity arriving in the aperture 35, which exits from aperture 32, will thus be

$$T2 = f2 * R_w / [1 - R_w * (1 - f1 - f2) - R1 * f1] ,$$

wherein f1 and f2 represent the ratio of the surface areas of aperture 32 and 35 to a surface area A of the entire cavity, Rw is a reflection factor for the diffusively reflective surface and R1 is a reflection factor for the outer end wall 35 or, more precisely, for the external surface 50 of the target 6. The value of the surface area A is not highly critical although a small value seems to beneficial in this case. The value given in this example is 100 mm$^2$. The reflection factor for the cavity was measured with the use of white silicone as Rw = 0,93 and the reflection factor for a taget, i.e. the human skin, was estimated, as measured with red light, to be R1 = 0,6. Thus, the reflectivity of the target 6 is generally also contributing to the signal. Using these values, the resulting transmission will be T2 = 0,13. A surface of the target 6 defined by the aperture 35 contains diffusively transmitting Lambert sources (7/2)$^2$ = 12,25 times more than what could be directly accommodated by the aperture 32, i.e. a higher number of sources in terms of the ratios between surface areas of the apertures. Thus, the gain of collection would be 0,13*12,25 = 1,59 i.e. the result will be a signal more than one and a half times stronger than what could be produced by collecting directly with the fiber end. The performed measurements have clearly supported this theory, although the augmentation of a signal has been slightly lesser than calculated, probably due to an imperfect contact between target 6 and the outer end wall of cavity 18, a lower-than-expected reflection factor for the target, and inhomogeneous lighting of the target.

Thus, with a larger surface area of the outer end wall 16, 26 and 35 it is possible to collect more light on the inner end wall 13a, 13b, 13c, 19a, 19b and 32 by using the diffusively reflective surface 14, 17, 21-23, 30 between the target 6 and a radiation source or a detector, e.g. in the cavity 18, 41, and at the same time the direction of radiation coming from the target can be deflected as desired or, in this case, to comply with the direction of the optical fiber 4, 31. Other benefits from using a larger surface area include a reduced sensitivity to a motion-related artefact, be it a directly external disturbance or an internal disturbance resulting for example from volumetric changes in venous blood.

The foregoing has described but a few examples of how a diffusively reflective surface or material can be utilized in a pulseoximeter sensor operating on fiberoptics. It is obvious that there are other conceivable types of solutions based on the same principle of diffusive reflection. The optical fiber is not absolutely necessary for proper functioning of the inventive principle, although a fiberoptic pulseoximeter sensor is a preferred application. Even a conventional sensor, V wherein light sources 38 and a detector 37 are included in a very sensor 100 closer to a target 6 to be measured, can be improved by using diffusively reflective surfaces in accordance with the invention. One such solution is depicted in fig. 6. In this solution, a detector 37 is located close to a target 6 to be measured, as in traditional sensor designs. On the other hand, the light sources 38, normally two diodes (LED) emitting at different wavelengths, are, according to the invention, mounted on an inner end wall 13b included in a diffusively reflective cavity 15c. In principle, the number of radiation sources 38 can be more than two, if so required by a particular application, or a more-than-normal amount of power can be supplied thereto. A higher light supply improves the signal-to-noise ratio, which otherwise may be too poor in certain operating conditions. The increased power consumption and the accompanying rise in temperature are not in this assembly focused on a target 6 to be measured but can be effectively guided to a top unit 1. This serves to eliminate possible heat-inflicted damages in the target 6 to be measured. In addition, the effect on a measuring result due to the position of various light sources will be equalized. Electric supply signals from both the detector 37 and the light sources 38 are carried along an electric cable 39 to a control monitor.

Fig. 7 illustrates another corresponding solution, wherein a diffusively reflective surface 14 included in a cavity 41 is not laterally curving but, instead, a direct cone 15a facing straight towards a target 6 and having its inner end wall 13b, along with radiation sources 38 included therein, at such a distance from the target 6 to be measured that eventual generated heat can be delivered away from the target to a frame 1. The cavity-forming direct cone 15a has a function otherwise similar to slanting or arching funnels 15b and 15c except that the proportion of direct light from sources to target is higher. If the funnels 15a-c reflect diffusively at a high efficiency, the signal should indicate no difference between the different types of cavities 18 and 41.

The detector may also utilize a diffusively reflective cavity for example for eliminating the effect of a motion-related artefact, as in the solution shown in fig. 8. In this solutions, light sources 38 are located close to the surface of a target 6 but could just as well be located further away from a target according to fig. 6 or 7. A detector 37 is further away from the surface of the target 6 at an inner end 19b included in a cavity, which is provided with a diffusively reflective surface and is, in this case, an arched funnel 15c. Exactly the same way as in the solution of fig. 7, the detector 37 may also be directed straight towards the target 6, as shown in fig. 9, by using an appropriately shaped direct cone 15a as a cavity 41. In the solutions shown in figs. 8 and 9, the detector 37 can be used for indicating light from a larger surface section of the target 6 and, as pointed out above, for producing even more signal than could be achieved if the detector were in a conventional manner close to the target surface.

All the above pulseoximeter sensors have been ones that are based on a transmission measured straight through a target. Thus, a direction S of the principal radiation from a radiation source 38 and/or a respective diffusively reflective surface and a principal sensitivity direction H indicated by way of a detector and/or a respective diffusively reflective surface are substantially opposite to each other, i.e. form a relative angle of 180° or form an obtuse angle V1, as illustrated in fig. 13. This produces a light 54 passing directly through a target for obtaining a measuring signal.

There is nothing to stop applying the inventive principle also to a pulseoximeter sensor 101 operating on a reflection principle. In this type of preferred sensor configuration, as shown e.g. in fig. 10, it is important that light sources 38 and a detector 37 be located side by side in the direction of an external target surface 50 at such a distance from each other that a light 44 is forced to progress an arterial-blood containing tissue of a target 6 to be measured prior to reflecting back instead of traveling along the surface layer of a tissue. A cavity 41, including a diffusively reflective surface and having its inner end wall 13c provided with a detector 37, is also in this type of sensor capable of collecting more light from deep in the tissue than what could be achieved if the detector were in a direct contact with the target 6. The sensor assembly is generally provided with just one body element 42 and a pad 43, sometimes combined, on which light sources and a detector are mounted and from which a signal is delivered for further processing by way of a cable 39. The sensor 101 can be fastened to a target by means of a tape, a rubber band or by other respective means. Although the described sensor is electric, there is nothing that would prevent the use of optical fibers for this solution as well, as already explained above. The cavity 41, shown in fig. 10 and including a diffusively reflective surface, only measures light over one side of the light sources. The aperture in the cavity 41, opening towards a target 6, need not be circular as some other appropriate shape is equally suitable. Fig. 11 depicts a configuration for radiation sources 38 effectively collecting radiation reflected from the entire environment. In this solution, the light sources 38 are located in the middle of an outer end 26 included in the cavity 41 provided with a diffusively reflective surface and at least close to the plane of said outer end, in other words, the light sources and detectors are fitted within each other. Thus, the detector 37, which is located in alignment with an inner end 13c of the cavity, receives reflected light 44 from as large as possible a target surface area. The light sources 38 are optically insulated from the cavity 41 with a light blocking surface 45, which surrounds the light sources in the direction of the outer end, whereby the light being collected in the cavity has inevitably been forced to travel deep in the tissue. In this case, the cavity 41 is preferably filled with a transparent material, such as silicone. In the embodiments of figs. 10 and 11, a direction S of the principal radiation from a radiation source 38 and/or a respective diffusively reflective surface and a principal sensitivity direction H indicated by way of a detector and/or a respective diffusively reflective surface are substantially co-directional, i.e. form a relative angle of 0° or form an acute angle V2, as illustrated in fig. 14. This produces a light 44 reflecting from the tissue of a target for obtaining a measuring signal.

The disclosed embodiments are but examples of how a diffusively reflective cavity can be used in a pulseoximeter sensor. It is self-evident that other representative solutions exist as well. For example, it is conceivable to use just a single light source and respectively two or more detectors, although the above-described configurations are most common.

Thus, it is not a requirement in this invention that both the radiation sources 38 and the detectors 37 be provided with diffusively reflective surfaces but either one is nevertheless equipped with a diffusively reflective surface fitted between the external target surface 50 and a radiation source or a detector. Conditions permitting, the sensor may only be provided with one above-mentioned funnel, either on the inlet or outlet side, which provides a diffusively reflective surface. A second funnel is then replaced by another type of construction, such as electronic components or a different fiberoptic system. In principle, the funnel may be nearly any shape at all. Fig. 2 illustrates a slanting cone 15b, figs. 3, 6 and 8 depict an arched cone 15c, and figs. 7-8 and 10-11 show a direct cone 15a. The slanting wedge illustrated in conjunction with figs. 5A-5B is a special case among these funnels. The funnel, which forms a cavity 18, 41, need not be diffusively reflective over its entire inner surface, although it is most preferred. If necessary, it is possible to employ such a diffusively reflective surface, towards which the sensitive surface of detectors and/or respectively the emitting surface of radiation sources and/or the end of an optical fiber are directed. The rest of the interior of an eventual cavity 18, 41 can be made of another type of material, e.g. some more or less radiation-absorbing material. For example, if in the case of figs. 5A-5B, the diffusively reflective surface 30 is flat and orthogonal to the image plane, the ends not shown in the figures and extending parallel to the image plane can be non-diffusively reflective. However, it is preferred that as large a portion as possible of a radiation transfer section fitted between the inner end and the outer end be designed as diffusively reflective according to the invention.

## Claims

1. A non-invasive optical sensor suitable for measuring the content or composition of one or several chemical component within a body portion of a patient, said sensor (100, 101) comprising:

   - a radiation source or radiation sources (38) for emitting measuring radiation with at least two wavelengths to the portion of the patient's body;
   - detectors or a detector (37) for receiving measuring radiation transmitted through said body portion of a patient and for providing respective electrical signal(s);
   - at least one radiation transfer section having a first end aperture (16; 26; 35) adapted to face an external surface (50) of said body portion (6), and a second end aperture (13 a,b,c; 19a,b; 32) facing said detector(s) or said radiation source(s) or optical fibers (31),

   characterized in that said first end aperture (16; 26; 35) has a surface area at least double with respect to that of said second end aperture (13 a,b,c; 19a,b; 32); and that said radiation transfer section further comprises a diffusively reflecting surface (14, 17; 21-23; 30), having properties approaching those of a Lambertian surface, between these end apertures for receiving radiation from one of said end apertures and transferring same to the other of said end apertures, and providing a generally uniform scattering of said received radiation.

2. A non-invasive optical sensor of claim 1, characterized in that said diffusively reflective surface (14, 17; 21-23; 30) is a portion in a jacket of a cavity (18, 41), which is in the shape of a direct (15a), slanting (15b) or arched (15c) funnel, extending from said first end aperture to said second end aperture and having a circular, elliptical or polygonal cross-sectional shape; that the second end aperture (13a,b,c; 19a,b; 32) and the first end aperture (16; 26; 35) are either parallel to each other or form an angle (K) relative to each other; and that said area of the first end aperture is tenfold with respect to said area of the second end aperture.

3. A non-invasive optical sensor of claim 2, characterized in that said diffusively reflective surface forms at least the main part of said cavity (18, 41); or alternatively said funnel is a symmetric or asymmetric wedge, wherein a first wedge surface (30) is substantially orthogonal to the bisector of an angle formed by the normals of said first and second end apertures (32 and 35) with each other, and at least this first wedge surface (30) is diffusively reflective.

4. A non-invasive optical sensor of claim 2, charac-

**terized in that**, when said funnel is an arched cone (15c), said first and second end apertures are located such that the normals thereof intersect the diffusively reflective surface (14, 17) or an extension thereof on the side of the cone with a maximum radius of curvature.

5. A non-invasive optical sensor of claim 1 or 2, **characterized in that** said first and second end apertures form a relative angle (K), which is at least 45°, or between 60°- 95°.

6. A non-invasive optical sensor suitable for measuring the content or composition of one or several chemical component within a body portion of a patient, said sensor (100, 101) comprising:

    - a radiation source or radiation sources (38) for emitting measuring radiation with at least two wavelengths to the portion of the patient's body;
    - detectors or a detector (37) for receiving measuring radiation transmitted through said body portion of a patient and for providing respective electrical signal(s);
    - at least one radiation transfer section having a first end aperture (16; 26; 35) adapted to face an external surface (50) of said body portion (6), and a second end aperture (13 a,b,c; 19a, b; 32) facing said detector(s) or said radiation source(s) or optical fibers (31), and changing the radiation direction,

    **characterized in that** said first end aperture is larger than said second end aperture, said second end aperture (13 a,b,c; 19a,b; 32) forming relative to said first end aperture an angle (K), which is between 30°-100°, or between 60°-95°; and that said radiation transfer section further comprises a diffusively reflecting surface (14, 17; 21-23; 30), having properties approaching those of a Lambertian surface, between these end apertures for receiving radiation from one of said end apertures and transferring same to the other of said end apertures, and providing a generally uniform scattering of said received radiation.

7. A non-invasive optical sensor of claim 6, **characterized in that** said diffusively reflective surface (14, 17; 21-23; 30) is a portion in a jacket of a cavity (18, 41), which is: in the shape of a slanting funnel (15b) or an arched funnel (15c) extending from said first end aperture to said second end aperture and the major part thereof being diffusively reflective; or an asymmetric wedge, wherein a first wedge surface is substantially orthogonal to the bisector of an angle formed by the normals of the first and second end apertures relative to each other, and at least this first wedge surface (30) being diffusively reflective.

8. A non-invasive optical sensor of claim 1 or 6, **characterized in that** between said second end aperture (13a,b,c; 19a,b; 32) and the radiation sources (38) or the detector(s) (37) respectively is fitted a fiberoptic radiation conductor (2, 4), which consists of one or a plurality of optical fibers, and that the fiberoptic radiation conductor consists of glass fiber or some other long-wave infrared radiation transmitting single or a plurality of fibers.

9. A non-invasive optical sensor of claim 1 or 6, **characterized in that** the detector(s) (38) and/or the radiation source(s) (37) are located in line with said second end aperture (13a,b,c; 19a,b; 32) and that the radiation sensitive surfaces or respectively the emitting surfaces thereof or, alternatively, the ends of optical fibers are directed towards the diffusively reflective surface (14, 17; 21-23; 30).

10. A non-invasive optical sensor of claim 1 or 6, **characterized in that** said diffusively reflective surfaces (14, 17; 21-23; 30) scatter a main part (24; 25, 26; 27, 28) of arriving radiation intensity (20) in directions other than the mirror-reflection departing angle corresponding to the angle of incidence of arriving radiation.

11. A non-invasive optical sensor of claim 1 or 6, **characterized in that** said diffusively reflective surface (14, 17; 21-23; 30) consists of a solid material, provided with a cavity which is coated on the inside with a diffusively reflective surface material, or the diffusively reflective surface (14, 17; 21-23; 30) consists of a partially radiation-transmitting and simultaneously diffusively reflective material; and that a gap between said first end aperture and said second end aperture (13a,b,c; 19a,b; 32 and 16; 26; 35) is up to the diffusively reflective surface filled with a radiation transmitting material, such as an appropriate gas or a transparent silicone compound.

12. A non-invasive optical sensor of claim 1 or 6, **characterized in that** in this sensor:

    {A} the detector (37) with its associated diffusively reflective surfaces (17; 21-23; 30) and the radiation sources (38); or
    {B} the detector (37) and the radiation source (38) with its associated diffusively reflective surfaces (14; 21-23); or
    {C} the detector (37) with its associated diffusively reflective surfaces (17; 21-23; 30) and the radiation source (38) with its associated diffusively reflective surfaces (14; 21-23) are located either

- opposite to and substantially in register with each other, whereby a sensitivity direction (H) and a radiation direction (S) extend against each other or form together an obtuse angle (V1) creating a direct transmission (54), or
- side by side, whereby the sensitivity direction (H) and the radiation direction (S) extend in the same direction or form together an acute angle (V2) creating a reflection (44).

**13.** A non-invasive optical sensor of any of the preceding claims, **characterized in that** said sensor is connected with an pulse oximeter measuring apparatus.

**14.** A use of a non-invasive optical sensor suitable for measuring the content or composition of one or several chemical component within a body portion of a patient, said sensor comprising:

- a radiation source or radiation sources (38) for emitting measuring radiation with at least two wavelengths to the portion of the patient's body;
- detectors or a detector (37) for receiving measuring radiation transmitted through said body portion of a patient and for providing respective electrical signal(s);
- at least one radiation transfer section having a first end aperture (16; 26; 35) adapted to face an external surface (50) of said body portion (6), and a second end aperture (13 a,b,c; 19a, b; 32) facing said detector(s) or said radiation source(s) or optical fibers (31), and having properties for changing the direction and/or enlarging the application area of said radiation on said body surface, **characterized in that**:

- said transfer section is a cavity (18, 41) provided with a diffusively reflective surface (14, 17; 21-23;30) between the first end aperture (16; 26; 35) and the second end aperture (13a,b,c; 19a,b; 32) thereof for scattered radiation transmitting, and said first end aperture being larger than or forming an angle relative to said second end aperture;
- said first end apertures of said at least one radiation transfer section are set against the external body surface (50) of the patient for emitting and/or receiving said measuring radiation; and
- oxygen saturation of the patient's blood is measured.

**15.** A use of claim 14, **characterized in that** the measurement occurs on the basis of that radiation which directly passes (54) through the blood-containing portion (6) of a patient's body, whereby the detector with cavity and the radiation source, or the radiation source with cavity and the detector, or the detector with cavity and the radiation source with cavity are located opposite to each other and substantially in register with each other, the sensitivity direction (H) and the radiation direction (S) extending against each other or forming together an obtuse angle (V1).

**16.** A use of claim 14, **characterized in that** the measurement occurs on the basis of that radiation which reflects (44) from inside the blood-containing portion (6) of a patient's body, whereby the detector with cavity and the radiation source, or the radiation source with cavity and the detector, or the detector with cavity and the radiation source with cavity are located side by side or within each other, the sensitivity direction (H) and the radiation direction (S) extending in the same direction or forming together an acute angle (V2).

**17.** A use of claim 14, **characterized in that** the radiation source or radiation sources (38) are located by means of a fiberoptic radiation conductor (2) remotely from the sensor (100) and/or the detector or detectors (37) are located by means of a fiberoptic radiation conductor (4) remotely from the sensor (100).

**Patentansprüche**

**1.** Nicht-invasiver, optischer Sensor, welcher für ein Messen des Gehalts oder der Zusammensetzung von einer oder mehreren chemischen Komponente(n) innerhalb eines Körperabschnitts eines Patienten geeignet ist, wobei der Sensor (100, 101) umfaßt:

- eine Strahlungsquelle oder Strahlungsquellen (38) zum Emittieren von Meßstrahlung mit wenigstens zwei Wellenlängen zu dem Abschnitt des Körpers des Patienten;
- Detektoren oder einen Detektor (37) zum Empfangen von Meßstrahlung, welche durch den Körperabschnitt eines Patienten durchgelassen bzw. durchgeleitet wurde, und zum Bereitstellen eines bzw. von entsprechenden elektrischen Signals(en);
- wenigstens einen Strahlungsübertragungsquerschnitt bzw. -abschnitt, welcher eine erste Endöffnung (16; 26; 35), welche adaptiert ist, um zu einer äußeren Oberfläche (50) des Körperabschnitts gerichtet zu sein, und eine zweite Endöffnung (13a, b, c; 19a, b; 32) umfaßt, welche zu dem (den) Detektor(en) und der (den) Strahlungsquelle(n) oder optischen Fasern (31) gerichtet ist,

**dadurch gekennzeichnet, daß** die erste Endöffnung (16; 26; 35) einen Oberflächenbereich von wenigstens dem doppelten relativ zu demjenigen der zweiten Endöffnung (13a, b, c; 19a, b; 32) aufweist und daß der Strahlungsübertragungsquerschnitt weiters eine diffus reflektierende Oberfläche (14, 17; 21 - 23; 30), welche Eigenschaften aufweist, welche sich denjenigen einer Lambert'schen Oberfläche annähern, zwischen diesen Endöffnungen zum Empfangen einer Strahlung von einer der Endöffnungen und zum Übertragen derselben auf die andere der Endöffnungen und zum Bereitstellen einer im allgemeinen einheitlichen bzw. gleichförmigen Streuung der empfangenen Strahlung umfaßt.

2. Nicht-invasiver, optischer Sensor nach Anspruch 1, **dadurch gekennzeichnet, daß** die diffus reflektierende Oberfläche (14, 17; 21 - 23; 30) ein Abschnitt in einer Umhüllung bzw. Verkleidung eines Hohlraums (18, 41) ist, welche in der Form eines geraden (15a), geneigten (15b) oder gekrümmten (15c) Trichters vorliegt, welcher sich von der ersten Endöffnung zu der zweiten Endöffnung erstreckt und eine kreisförmige, elliptische oder polygonale Querschnittsform aufweist; daß die zweite Endöffnung (13a, b, c; 19a, b; 32) und die erste Endöffnung (16; 26; 35) entweder parallel zueinander sind oder einen Winkel (K) relativ zueinander ausbilden; und daß der Bereich der ersten Endöffnung das Zehnfache in bezug auf den Bereich bzw. die Fläche der zweiten Endöffnung beträgt.

3. Nicht-invasiver, optischer Sensor nach Anspruch 2, **dadurch gekennzeichnet, daß** die diffus reflektierende Oberfläche wenigstens den Hauptteil des Hohlraums (18, 41) bildet; oder alternativ der Trichter ein symmetrischer oder asymmetrischer Keil ist, worin eine erste Keiloberfläche (30) im wesentlichen orthogonal auf die Halbierende eines Winkels bzw. Winkelhalbierende ist, welche durch die Normalen der ersten und zweiten Endöffnung (32 und 35) miteinander gebildet wird, und wenigstens diese erste Keiloberfläche (30) diffus reflektierend ist.

4. Nicht-invasiver, optischer Sensor nach Anspruch 2, **dadurch gekennzeichnet, daß**, wenn der Trichter ein gekrümmter Konus (15c) ist, die erste und zweite Endöffnung derart angeordnet sind, daß die Normalen davon die diffus reflektierende Oberfläche (14, 17) oder eine Erstreckung bzw. einen Fortsatz davon an der Seite des Konus mit einem maximalen Krümmungsradius schneiden bzw. kreuzen.

5. Nicht-invasiver, optischer Sensor nach Anspruch 1 oder 2, **dadurch gekennzeichnet, daß** die erste und zweite Endöffnung einen relativen Winkel (K) ausbilden, welcher wenigstens 45° oder von 60° bis 95° beträgt.

6. Nicht-invasiver, optischer Sensor, welcher für ein Messen des Gehalts oder der Zusammensetzung von einer oder mehreren chemischen Komponente (n) innerhalb eines Körperabschnitts eines Patienten geeignet ist, wobei der Sensor (100, 101) umfaßt:

   - eine Strahlungsquelle oder Strahlungsquellen (38) zum Emittieren von Meßstrahlung mit wenigstens zwei Wellenlängen zu dem Abschnitt des Körpers des Patienten;
   - Detektoren oder einen Detektor (37) zum Empfangen von Meßstrahlung, welche durch den Körperabschnitt eines Patienten durchgelassen wurde, und zum Bereitstellen eines bzw. von elektrischen Signals(en);
   - wenigstens einen Strahlungsübertragungsquerschnitt bzw. -abschnitt, welcher eine erste Endöffnung (16; 26; 35), welche adaptiert ist, um zu einer externen Oberfläche (50) des Körperabschnitts gerichtet zu sein, und eine zweite Endöffnung (13a, b, c; 19a, b; 32) umfaßt, welche zu dem (den) Detektor(en) und der den Strahlungsquelle(n) oder optischen Fasern (31) gerichtet ist, und die Strahlungsrichtung ändert,

   **dadurch gekennzeichnet, daß** die erste Endöffnung größer ist als die zweite Endöffnung, wobei die zweite Endöffnung (13a, b, c; 19a, b; 32) relativ zu der ersten Endöffnung einen Winkel (K) ausbildet, welcher zwischen 30° und 100°, oder zwischen 60° und 95° liegt, und daß der Strahlungsübertragungsquerschnitt weiters eine diffus reflektierende Oberfläche (14, 17; 21 - 23; 30), welche Eigenschaften aufweist, welche sich denjenigen einer Lambert'schen Oberfläche annähern, zwischen diesen Endöffnungen zum Empfangen von Strahlung von einer der Endöffnungen und zum Übertragen derselben zu der anderen der Endöffnungen aufweist, und ein im allgemeinen einheitliches bzw. gleichmäßiges Streuen der empfangenen Strahlen zur Verfügung stellt.

7. Nicht-invasiver, optischer Sensor nach Anspruch 6, **dadurch gekennzeichnet, daß** die diffus reflektierende Oberfläche (14, 17; 21 - 23; 30) einen Abschnitt in einer Ummantelung eines Hohlraums (18, 41) ist, welcher in der Form eines geneigten bzw. sich verjüngenden Trichters (15b) oder eines gekrümmten bzw. gebogenen Trichters (15c) vorliegt, welcher sich von der ersten Endöffnung zu der zweiten Endöffnung erstreckt und von welchem der Hauptteil diffus reflektierend ist; oder eines asymmetrischen Keils vorliegt, worin eine erste Keiloberfläche im wesentlichen orthogonal auf die Winkelhalbierende eines Winkels ist, welcher durch die Normale der ersten und zweiten Endöffnung relativ zueinander gebildet wird, und wenigstens diese erste Keiloberfläche (30) diffus reflektierend ist.

8. Nicht-invasiver, optischer Sensor nach Anspruch 1 oder 6, **dadurch gekennzeichnet, daß** zwischen der zweiten Endöffnung (13a, b, c; 19a, b; 32) und den Strahlungsquellen (38) oder dem (den) Detektor(en) (37) jeweils ein faseroptischer Strahlungsleiter (2, 4) eingepaßt ist, welcher aus einer oder einer Vielzahl von optischen Fasern besteht, und daß der faseroptische Strahlungsleiter aus einer Glasfaser oder einigen anderen einzelnen, langwellige Infrarotstrahlung übertragenden Einzelfasern oder einer Vielzahl von Fasern besteht.

9. Nicht-invasiver, optischer Sensor nach Anspruch 1 oder 6, **dadurch gekennzeichnet, daß** der (die) Detektor(en) (38) und/oder die Strahlungsquelle(n) (37) in einer Linie mit der zweiten Endöffnung (13a, b, c; 19a, b; 32) angeordnet ist bzw. sind und daß die für eine Strahlung empfindlichen Oberflächen oder jeweils die emittierenden Oberflächen davon oder alternativ die Enden von optischen Fasern zu der diffus reflektierenden Oberfläche (14, 17; 21 - 23; 30) gerichtet sind.

10. Nicht-invasiver, optischer Sensor nach Anspruch 1 oder 6, **dadurch gekennzeichnet, daß** die diffus reflektierenden Oberflächen (14, 17; 21 - 23; 30) einen Hauptteil (24; 25, 26; 27, 28) von einlangender bzw. ankommender Strahlungsintensität (20) in Richtungen verschieden von dem Spiegelreflexions-Austrittswinkel entsprechend dem Einfallswinkel einer einlangenden Strahlung streuen.

11. Nicht-invasiver, optischer Sensor nach Anspruch 1 oder 6, **dadurch gekennzeichnet, daß** die diffus reflektierende Oberfläche (14, 17; 21 - 23; 30) aus einem festen Material besteht, welches mit einem Hohlraum versehen ist, welcher an der Innenseite mit einem diffus reflektierenden Oberflächenmaterial beschichtet ist, oder daß die diffus reflektierende Oberfläche (14, 17; 21 - 23; 30) aus einem teilweise eine Strahlung übertragenden bzw. durchlassenden und gleichzeitig diffus reflektierenden Material besteht; und daß ein Spalt zwischen der ersten Endöffnung und der zweiten Endöffnung (13a, b, c; 19a, b; 32 und 16; 26; 35) bis zu der diffus reflektierenden Oberfläche mit einem eine Strahlung transmittierenden bzw. durchlassenden Material, wie beispielsweise einem geeigneten Gas oder einer transparenten Silikonverbindung, gefüllt ist.

12. Nicht-invasiver, optischer Sensor nach Anspruch 1 oder 6, **dadurch gekennzeichnet, daß** in diesem Sensor:

{A} der Detektor (37) mit seinen zugehörigen, diffus reflektierenden Oberflächen (17; 21 - 23; 30) und den Strahlungsquellen (38); oder
{B} der Detektor (37) und die Strahlungsquelle (38) mit ihren zugehörigen, diffus reflektierenden Oberflächen (14; 21 - 23); oder
{C} der Detektor (37) mit seinen zugehörigen, diffus reflektierenden Oberflächen (17; 21 - 23; 30) und die Strahlungsquelle (38) mit ihren zugehörigen, diffus reflektierenden Oberflächen (14; 21 - 23) entweder

- gegenüberliegend zueinander und im wesentlichen miteinander ausgerichtet, wodurch eine Empfindlichkeitsrichtung (H) und eine Strahlungsrichtung (S) sich gegeneinander erstrekken oder gemeinsam einen stumpfen Winkel (V1) bilden, wodurch eine direkte Transmission bzw. Übertragung (54) erzeugt wird, oder
- nebeneinander angeordnet sind, wodurch die Empfindlichkeitsrichtung (H) und die Strahlungsrichtung (S) sich in derselben Richtung erstrecken oder einen spitzen Winkel (V2) miteinander bilden, welcher eine Reflexion (44) erzeugt.

13. Nicht-invasiver, optischer Sensor nach einem der vorangehenden Ansprüche, **dadurch gekennzeichnet, daß** der Sensor mit einer Pulsoximeter-Meßvorrichtung verbunden ist.

14. Verwendung eines nicht-invasiven, optischen Sensors, welcher für ein Messen des Gehalts oder der Zusammensetzung von einer oder mehreren chemischen Komponente(n) innerhalb eines Körperabschnitts eines Patienten geeignet ist, wobei der Sensor umfaßt:

- eine Strahlungsquelle oder Strahlungsquellen (38) zum Emittieren von Meßstrahlung mit wenigstens zwei Wellenlängen zu dem Abschnitt des Körpers des Patienten;
- Detektoren oder einen Detektor (37) zum Empfangen von Meßstrahlung, welche durch den Körperabschnitt eines Patienten durchgelassen wurde, und zum Bereitstellen eines bzw. von elektrischen Signals(en);
- wenigstens einen Strahlungsübertragungsquerschnitt bzw. -abschnitt, welcher eine erste Endöffnung (16; 26; 35), welche adaptiert ist, zu einer externen Oberfläche (50) des Körperabschnitts gerichtet zu sein, und eine zweite Endöffnung (13a, b, c; 19a, b; 32) umfaßt, welche zu dem (den) Detektor(en) und der (den) Strahlungsquelle(n) oder optischen Fasern (31) gerichtet ist, und Eigenschaften zum Ändern der Richtung und/oder Vergrößern des Anwendungsbereichs der Strahlung auf der Körperoberfläche aufweist, **dadurch gekennzeichnet, daß**:

- der Transfer- bzw. Übertragungsquerschnitt (18, 41) mit einer diffus reflektierenden Oberfläche (14, 17; 21 - 23; 30) zwischen der ersten Endöffnung (16; 26; 35) und der zweiten Endöffnung (13a, b, c; 19a, b; 32) davon für ein Übertragen von gestreuter Strahlung versehen ist, und worin die erste Endöffnung größer ist als die zweite Endöffnung oder einen Winkel relativ dazu bildet;
- die ersten Endöffnungen des wenigstens einer Strahlungsübertragungsquerschnitts gegen die äußere Körperoberfläche (50) des Patienten für ein Emittieren und/oder Empfangen der Meßstrahlen eingestellt bzw. festgelegt sind; und
- eine Sauerstoffsättigung des Bluts des Patienten gemessen wird.

**15.** Verwendung nach Anspruch 14, **dadurch gekennzeichnet, daß** die Messung auf der Basis der Strahlung auftritt, welche direkt (54) durch den Blut enthaltenden Abschnitt (6) eines Patientenkörpers hindurchtritt, wobei der Detektor mit dem Hohlraum und der Strahlungsquelle oder die Strahlungsquelle mit dem Hohlraum und der Detektor, oder der Detektor mit dem Hohlraum und die Strahlungsquelle mit dem Hohlraum gegenüberliegend zueinander angeordnet sind und im wesentlichen miteinander ausgerichtet sind, wobei sich die Empfindlichkeitsrichtung (H) und die Strahlungsrichtung (S) gegeneinander erstrecken oder einen stumpfen Winkel (V1 ) miteinander bilden.

**16.** Verwendung nach Anspruch 14, **dadurch gekennzeichnet, daß** die Messung auf der Basis der Strahlung auftritt, welche von der Innenseite des Blut enthaltenden Abschnitts (6) eines Patientenkörpers reflektiert wird (44), wobei der Detektor mit dem Hohlraum und die Strahlungsquelle, oder die Strahlungsquelle mit dem Hohlraum und der Detektor, oder der Detektor mit dem Hohlraum und die Strahlungsquelle mit dem Hohlraum nebeneinander oder ineinander angeordnet sind, wobei sich die Empfindlichkeitsrichtung (H) und die Strahlungsrichtung (S) in derselben Richtung erstrecken oder miteinander einen spitzen Winkel (V2) ausbilden.

**17.** Verwendung nach Anspruch 14, **dadurch gekennzeichnet, daß** die Strahlungsquellen oder die Strahlungsquellen (38) mit Hilfe eines faseroptischen Strahlungsleiters (2) entfernt von dem Sensor (100) angeordnet ist bzw. sind und/ oder der Detektor oder die Detektoren (37) mit Hilfe eines faseroptischen Strahlungsleiters (4) entfernt von dem Sensor (100) angeordnet ist bzw. sind.

**Revendications**

**1.** Capteur optique non invasif convenant pour la mesure du contenu ou de la composition d'un ou de plusieurs composants chimiques à l'intérieur d'une partie de corps d'un patient, ledit capteur (100, 101) comprenant :

une source ou des sources de rayonnement (38) pour émettre un rayonnement de mesure moyennant au moins deux longueurs d'onde sur la partie du corps de patient ; des détecteurs ou un détecteur (37) pour recevoir un rayonnement de mesure qui est transmis au travers de ladite partie de corps d'un patient et pour appliquer un signal ou des signaux électriques respectifs ; au moins une section de transfert de rayonnement qui comporte une première ouverture d'extrémité (16 ; 26 ; 35) qui est adaptée de manière à faire face à une surface externe (50) de ladite partie de corps (6) et une seconde ouverture d'extrémité (13a, b, c ; 19a, b ; 32) qui fait face audit détecteur ou auxdits détecteurs ou à ladite source ou auxdites sources de rayonnement ou à des fibres optiques (31), **caractérisé en ce que** ladite première ouverture d'extrémité (16 ; 26 ; 35) présente une aire de surface qui est au moins le double de celle de ladite seconde ouverture d'extrémité (13a, b, c ; 19a, b ; 32) ; et **en ce que** ladite section de transfert de rayonnement comprend en outre une surface réfléchissante de façon diffuse (14, 17 ; 21-23 ; 30) présentant des propriétés qui s'approchent de celles d'une surface lambertienne, entre ces ouvertures d'extrémité pour recevoir un rayonnement depuis l'une desdites ouvertures d'extrémité et pour transférer ce même rayonnement sur l'autre desdites ouvertures d'extrémité, et pour assurer une diffusion généralement uniforme dudit rayonnement reçu.

**2.** Capteur optique non invasif selon la revendication 1, **caractérisé en ce que** ladite surface réfléchissante de façon diffuse (14, 17 ; 21-23 ; 30) est une partie dans une enveloppe d'une cavité (18, 41), qui est selon la forme d'un entonnoir direct (15a), incliné (15b) ou arqué (15c) qui s'étend depuis ladite première ouverture d'extrémité jusqu'à ladite seconde ouverture d'extrémité et qui présente une forme en coupe circulaire, elliptique ou polygonale ; **en ce que** la seconde ouverture d'extrémité (13a, b, c ; 19a, b ; 32) et la première ouverture d'extrémité (16 ; 26 ; 35) soit sont parallèles l'une à l'autre, soit forment un angle (K) l'une par rapport à l'autre ; et **en ce que** ladite aire de la première ouverture d'extrémité est d'un facteur 10 par rapport à ladite aire de la seconde ouverture d'extrémité.

3. Capteur optique non invasif selon la revendication 2, **caractérisé en ce que** ladite surface réfléchissante de façon diffuse forme au moins la partie principale de ladite cavité (18, 41) ; ou selon une variante, ledit entonnoir est un coin symétrique ou asymétrique, dans lequel une première surface de coin (30) est sensiblement orthogonale à la bissectrice d'un angle qui est formé par les normales auxdites première et seconde ouvertures d'extrémité (32 et 35), et au moins cette première surface de coin (30) est réfléchissante de façon diffuse.

4. Capteur optique non invasif selon la revendication 2, **caractérisé en ce que**, lorsque ledit entonnoir est un cône arqué (15c), lesdites première et seconde ouvertures d'extrémité sont localisées de telle sorte que leurs normales intersectent la surface réfléchissante de façon diffuse (14, 17) ou une extension de celle-ci sur le côté du cône moyennant un rayon de courbure maximum.

5. Capteur optique non invasif selon la revendication 1 ou 2, **caractérisé en ce que** lesdites première et seconde ouvertures d'extrémité forment un angle relatif (K) qui vaut au moins 45° ou qui est compris entre 60° et 95°.

6. Capteur optique non invasif convenant pour la mesure du contenu ou de la composition d'un ou de plusieurs composants chimiques à l'intérieur d'une partie de corps d'un patient, ledit capteur (100, 101) comprenant :

> une source ou des sources de rayonnement (38) pour émettre un rayonnement de mesuré moyennant au moins deux longueurs d'onde sur la partie du corps de patient ;
> des détecteurs ou un détecteur (37) pour recevoir un rayonnement de mesure qui est transmis au travers de ladite partie de corps d'un patient et pour appliquer un signal ou des signaux électriques respectifs ;
> au moins une section de transfert de rayonnement qui comporte une première ouverture d'extrémité (16 ; 26 ; 35) qui est adaptée de manière à faire face à une surface externe (50) de ladite partie de corps (6) et une seconde ouverture d'extrémité (13a, b, c ; 19a, b ; 32) qui fait face audit détecteur ou auxdits détecteurs ou à ladite source ou auxdites sources de rayonnement ou à des fibres optiques (31), et qui modifie la direction de rayonnement, **caractérisé en ce que** ladite première ouverture d'extrémité est plus grande que ladite seconde ouverture d'extrémité, ladite seconde ouverture d'extrémité (13a, b, c ; 19a, b ; 32) formant, par rapport à ladite première ouverture d'extrémité, un angle (K) qui est compris entre 30° et 100° ou entre 60° et 95° ; et **en ce que** ladite section de transfert de rayonnement comprend en outre une surface réfléchissante de façon diffuse (14, 17 ; 21-23 ; 30) qui présente des propriétés qui s'approchent de celles d'une surface lambertienne, entre ces ouvertures d'extrémité pour recevoir un rayonnement depuis l'une desdites ouvertures d'extrémité et pour transférer ce même rayonnement sur l'autre desdites ouvertures d'extrémité, et pour assurer une diffusion généralement uniforme dudit rayonnement reçu.

7. Capteur optique non invasif selon la revendication 6, **caractérisé en ce que** ladite surface réfléchissante de façon diffuse (14, 17 ; 21-23 ; 30) est une partie dans une enveloppe d'une cavité (18, 41), qui est selon la forme d'un entonnoir incliné (15b) ou arqué (15c) qui s'étend depuis ladite première ouverture d'extrémité jusqu'à ladite seconde ouverture d'extrémité et sa partie principale est réfléchissante de façon diffuse ; ou un coin asymétrique, dans lequel une première surface de coin (30) est sensiblement orthogonale à la bissectrice d'un angle qui est formé par les normales aux première et seconde ouvertures d'extrémité, et au moins cette première surface de coin (30) est réfléchissante de façon diffuse.

8. Capteur optique non invasif selon la revendication 1 ou 6, **caractérisé en ce que**, entre ladite seconde ouverture d'extrémité (13a, b, c ; 19a, b ; 32) et les sources de rayonnement (38) ou le ou les détecteurs (37), de façon respective, est adapté un conducteur de rayonnement à fibres optiques (2, 4) qui est constitué par une ou plusieurs fibres optiques et **en ce que** le conducteur de rayonnement à fibres optiques est constitué par une fibre de verre ou par une quelconque autre unique ou de quelconques autres plusieurs fibres transmettant un rayonnement infrarouge d'onde longue.

9. Capteur optique non invasif selon la revendication 1 ou 6, **caractérisé en ce que** le ou les détecteurs (38) et/ou la ou les sources de rayonnement (37) sont localisés en ligne avec ladite seconde ouverture d'extrémité (13a, b, c ; 19a, b ; 32) et **en ce que** les surfaces sensibles au rayonnement ou leurs surfaces émettrices, de façon respective, ou selon une variante, les extrémité de fibres optiques sont dirigées en direction de la surface réfléchissante de façon diffuse (14, 17 ; 21-23 ; 30).

**10.** Capteur optique non invasif selon la revendication 1 ou 6, **caractérisé en ce que** lesdites surfaces réfléchissantes de façon diffuse (14, 17 ; 21-23 ; 30) diffusent une partie principale (24 ; 25, 26 ; 27, 28) d'une intensité de rayonnement d'arrivée (20) suivant des directions autres que l'angle de sortie de réflexion miroir correspondant à l'angle d'incidence d'une rayonnement d'arrivée.

**11.** Capteur optique non invasif selon la revendication 1 ou 6, **caractérisé en ce que** ladite surface réfléchissante de façon diffuse (14, 17 ; 21-23 ; 30) est constituée par un matériau solide muni d'une cavité qui est revêtue sur l'intérieur d'un matériau de surface réfléchissant de façon diffuse, ou la surface réfléchissante de façon diffuse (14, 17 ; 21-23 ; 30) est constituée par un matériau transmettant partiellement le rayonnement et simultanément réfléchissant de façon diffuse ; et **en ce qu'**un espace entre ladite première ouverture d'extrémité et ladite seconde ouverture d'extrémité (13a, b, c ; 19a, b ; 32 et 16 ; 26 ; 35) va jusqu'à la surface réfléchissante de façon diffuse et est rempli d'un matériau transmettant le rayonnement tel qu'un gaz approprié ou qu'un composé de silicone transparent.

**12.** Capteur optique non invasif selon la revendication 1 ou 6, **caractérisé en ce que**, dans ce capteur :

{A} le détecteur (37) avec ses surfaces réfléchissantes de façon diffuse associées (17 ; 21-23 ; 30) et les sources de rayonnement (38) ; ou

{B} le détecteur (37) et la source de rayonnement (38) avec ses surfaces réfléchissantes de façon diffuse associées (14 ; 21-23) ; ou

{C} le détecteur (37) avec ses surfaces réfléchissantes de façon diffuse associées (17 ; 21-23 ; 30) et la source de rayonnement (38) avec ses surfaces réfléchissantes de façon diffuse associées (14 ; 21-23)

sont localisés :

- soit à l'opposé les uns des autres et sensiblement en repérage les uns avec les autres et ainsi, une direction de sensibilité (H) et une direction de rayonnement (S) s'étendent en opposition l'une par rapport à l'autre ou forment ensemble un angle obtus (V1) qui crée une transmission directe (54),

- soit côte à côte et ainsi, la direction de sensibilité (H) et la direction de rayonnement (S) s'étendent suivant la même direction ou forment ensemble un angle aigu (V2) qui crée une réflexion (44).

**13.** Capteur optique non invasif selon l'une quelconque des revendications précédentes, **caractérisé en ce que** ledit capteur est connecté à un appareil de mesure d'oxymètre par impulsion.

**14.** Utilisation d'un capteur optique non invasif qui convient pour mesurer le contenu ou la composition d'un ou de plusieurs composants chimiques à l'intérieur d'une partie de corps d'un patient, ledit capteur comprenant :

une source ou des sources de rayonnement (38) pour émettre un rayonnement de mesure moyennant au moins deux longueurs d'onde sur la partie du corps de patient ;
des détecteurs ou un détecteur (37) pour recevoir un rayonnement de mesure qui est transmis au travers de ladite partie de corps d'un patient et pour appliquer un signal ou des signaux électriques respectifs ;
au moins une section de transfert de rayonnement qui comporte une première ouverture d'extrémité (16 ; 26 ; 35) adaptée de manière à faire face à une surface externe (50) de ladite partie de corps (6) et une seconde ouverture d'extrémité (13a, b, c ; 19a, b ; 32) faisant face audit détecteur ou auxdits détecteurs ou à ladite source de rayonnement ou auxdites sources de rayonnement ou à des fibres optiques (31) et présentant des propriétés pour modifier la direction et/ou pour agrandir l'aire d'application dudit rayonnement sur ladite surface de corps,

**caractérisée en ce que** :

ladite section de transfert est une cavité (18, 41) qui est munie d'une surface réfléchissante de façon diffuse (14, 17 ; 21-23 ; 30) entre la première ouverture d'extrémité (16 ; 26 ; 35) et la seconde ouverture d'extrémité (13a, b, c ; 19a, b ; 32) afférentes pour une transmission de rayonnement diffusé, et ladite première ouverture d'extrémité est plus grande que ladite seconde ouverture d'extrémité ou forme un angle par rapport à cette même dite seconde ouverture d'extrémité ;
lesdites premières ouvertures d'extrémité de ladite au moins une section de transfert de rayonnement sont établies contre la surface de corps externe (50) du patient pour émettre et/ou recevoir ledit rayonnement de mesure ; et une saturation en oxygène du sang du patient est mesurée.

**15.** Utilisation selon la revendication 14, **caractérisée en ce que** la mesure est réalisée sur la base du rayonnement qui passe directement (54) au travers de la partie contenant du sang (6) d'un corps de patient et ainsi, le détecteur avec une cavité et la source de rayonnement ou la source de rayonnement avec une cavité et le détecteur ou le détecteur avec une cavité et la source de rayonnement avec une cavité sont localisés à l'opposé l'un de l'autre et sont sensiblement en repérage l'un avec l'autre, la direction de sensibilité (H) et la direction de rayonnement (S) s'étendant à l'opposé l'une de l'autre ou formant ensemble un angle obtus (VI).

16. Utilisation selon la revendication 14, **caractérisée en ce que** la mesure est réalisée sur la base du rayonnement qui est réfléchi (44) depuis l'intérieur de la partie contenant du sang (6) d'un corps de patient et ainsi, le détecteur avec une cavité et la source de rayonnement ou la source de rayonnement avec une cavité et le détecteur ou le détecteur avec une cavité et la source de rayonnement avec une cavité sont localisés côte à côte ou à l'intérieur l'un de l'autre, la direction de sensibilité (H) et la direction de rayonnement (S) s'étendant suivant la même direction ou formant ensemble un angle aigu (V2).

17. Utilisation selon la revendication 14, **caractérisée en ce que** la source de rayonnement ou les sources de rayonnement (38) sont localisées au moyen d'un conducteur de rayonnement à fibres optiques (2) à distance du capteur (100) et/ou le détecteur ou les détecteurs (37) sont localisés au moyen d'un conducteur de rayonnement à fibres optiques (4) à distance du capteur (100).

Fig. 1

Fig. 2

Fig. 3

Fig. 4

Fig. 5A

Fig. 5B

Fig. 6

Fig. 7

Fig. 8

Fig. 9

Fig. 10

Fig. 11

Fig. 12

Fig. 13

Fig. 14